# EUROPÄISCHE PATENTSCHRIFT

(11) **EP 0 381 049 B1**
(45) Veröffentlichungstag und Bekanntmachung des Hinweises auf die Patenterteilung: **09.06.1993**
(21) Anmeldenummer: 90101467.0
(22) Anmeldetag: 25.01.1990
(51) Int. Cl.: C07C 315/00, C07C 317/14

(54) **Verfahren zur Herstellung von Bis-(4-chlorphenyl)-sulfon**
Process for the preparation of bis-4-chlorophenyl sulfone
Procédé pour la préparation de bis-4-chlorophényl sulfone

(30) Priorität: 01.02.1989 DE 3902890
(43) Veröffentlichungstag der Anmeldung: 08.08.1990
(73) Patentinhaber: BASF Aktiengesellschaft, 67063 Ludwigshafen (DE)
(72) Erfinder: Stumpp, Michael, Dr., D-6705 Deidesheim (DE); Neumann, Peter, Dr., D-6800 Mannheim 31 (DE); Eilingsfeld, Heinz, Dr., D-6710 Frankenthal (DE)

(56) Entgegenhaltungen:
- EP-A- 0 279 388
- BE-A- 699 725
- DE-B- 1 087 592

## Beschreibung

Diese Erfindung betrifft ein verbessertes Verfahren zur Herstellung von Bis-(4-chlorphenyl)sulfon.

Bis-(4-chlorphenyl)-sulfon ist ein wichtiges Zwischenprodukt, das hauptsächlich für die Herstellung aromatischer Polysulfone und zur Synthese von Bis-(4-aminophenyl)-sulfon, das man sowohl für die Lepratherapie als auch zur Härtung von Epoxidharzen benötigt, verwendet wird. Voraussetzung für diese Einsatzgebiete ist eine hohe Reinheit des Bis-(4-chlorphenyl)-sulfons.

Bei den bekannten Verfahren zur Herstellung von Bis-(4-chlorphenyl)-sulfon wird Chlorbenzol in der Regel einer Sulfonierungsreaktion unterworfen. Da Chlorbenzol ein relativ reaktionsträger Partner für Sulfonierungsreaktionen ist, braucht man für einen guten Umsatz entweder reaktionsfähige Sulfonierungsmittel oder aber drastische Reaktionsbedingungen.

Aus der DE-A-10 87 592 ist ein Verfahren zur Herstellung von Bis-(4-chlorphenyl)-sulfon durch Umsetzung von Chlorbenzol mit einer Mischung aus Schwefeltrioxid und Dimethyl- oder Diethylpyrosulfat bekannt. Danach wird zunächst in einer getrennten Reaktion aus Dimethylsulfat und Schwefeltrioxid das Dimethylpyrosulfat und durch Zugabe einer weiteren Menge an Schwefeltrioxid der eigentliche reaktive Sulfonierungskomplex gebildet. Dieser wird dann in einer Folgereaktion in einem zusätzlichen Reaktionsgefäß mit Chlorbenzol zur Reaktion gebracht.

Nachteilig ist hierbei die zweistufige Verfahrensweise.

Der Erfindung lag daher die Aufgabe zugrunde ein verbessertes, einfacheres Verfahren zur Herstellung von Bis-(4-chlorphenyl)-sulfon durch Umsetzung von Chlorbenzol mit einem reaktionsfähigen Sulfonierungsmittel zu finden, das dem vorgenannten Nachteil abhilft.

Demgemäß wurde ein Verfahren zur Herstellung von Bis-(4-chlorphenyl)-sulfon gefunden, welches dadurch gekennzeichnet ist, daß man bei 50 bis 100°C Schwefeltrioxid, Dimethylsulfat und Chlorbenzol gleichzeitig zur Reaktion bringt.

Das erfindungsgemäße Verfahren kann wie folgt durchgeführt werden:

Das erfindungsgemäße Verfahren kann im chargenweisen Betrieb in Rührkesseln oder vorzugsweise kontinuierlich, beispielsweise in Rohr- oder Röhrenreaktoren ausgeübt werden. Die Verweilzeit der Reaktionslösung im Reaktor kann u.a. durch die Beschickung des Rohrreaktors mit Füllkörpern gesteuert werden, d.h. durch die Änderung der Geometrie, Korngröße und Packungsdichte der Füllkörper kann die Verweilzeit der Reaktionslösung im Reaktor erhöht oder erniedrigt werden. Bevorzugt ist dabei die Anwendung kugelförmiger Füllkörper, wie sie auch in Füllkörperkolonnen zur destillativen Auftrennung von Gemischen benutzt werden, beispielsweise Glaskugeln oder Kugeln aus gegenüber der Reaktionsmischung inerten Stählen. Der Durchmesser dieser Kugeln beträgt in der Regel 0,4 bis 10 mm, vorzugsweise 1 bis 5 mm. Zweckmäßigerweise werden Kugeln einer bestimmten Größe verwendet.

Vorteilhafterweise werden die Reaktorrohre mit Füllkörpern aus Materialien bepackt, welche die Sulfonbildung katalytisch beeinflussen und somit als Sulfonierungskatalysatoren wirken. Beispielsweise lassen sich die Ausbeute und die Selektivität der Umsetzung verbessern, wenn gesinterte Borsäurekugeln oder von mit einer Säure, wie Phosphor-, Schwefel- oder Borsäure, beladenes Kieselgel als Füllkörper eingesetzt werden. Derart modifiziertes Kieselgel wird zweckmäßigerweise durch die Imprägnierung von Kieselgel mit vorzugsweise wäßrigen Lösungen der betreffenden Säuren und anschließende Trocknung hergestellt. Die Borsäurekugeln werden im allgemeinen bei Temperaturen von 150 bis 200°C gesintert.

Bei der Verwendung von Glaskugeln mit einem Durchmesser von 4 mm betrug die anfängliche Verweilzeit ca. 1 Minute. Bei mehrmaligem Umpumpen und steigendem Umsatz wurde die Reaktionslösung viskoser und die durchschnittliche Verweilzeit erhöhte sich auf 3 Minuten.

Die Reaktionstemperatur beträgt in der Regel 50 bis 100, vorzugsweise 70 bis 90°C. Temperaturen unterhalb 50 °C sind zwar möglich, aber im Hinblick auf die Reaktionszeiten und die Umsätze nicht geboten. Vorteilhafterweise sollte die Reaktionstemperatur über 60 °C liegen. Werden Temperaturen über 100 °C eingestellt, so ist durch eine entsprechende Auswahl der Füllkörper oder durch eine Pumpe dafür zu sorgen, daß keine zu hohen Verweilzeiten im Reaktor auftreten. Es wurde festgestellt, daß bei Reaktionstemperaturen oberhalb 80 °C die Mengen an isomeren Dichlordiphenylsulfonen, vor allem aber an 3,4'-Bis(chlorphenyl)-sulfon erheblich zunehmen. So wurde bei 90°C über 10 % des unerwünschten 3,4'-Isomeren gefunden.

Das Molverhältnis von Schwefeltrioxid zu Dimethylsulfat zu Chlorbenzol liegt in aller Regel zwischen 0,01:0,005:1 und 2:1:1, vorzugsweise zwischen 0,1:0,05:1 und 1,5:0,8:1, besonders bevorzugt zwischen 0,5:0,25:1 und 1,1:0,6:1 und ist nicht sehr sensitiv bezüglich Abweichungen von der Standardzusammensetzung 1:0,5:1.

Bei einfachem Durchlauf der Komponenten durch den Reaktor und Verweilzeiten von 1 bis 3 Minuten wurde ein Gesamtumsatz von 57 % erzielt. Der Gesamtumsatz kann auf 91 % gesteigert werden, wenn man das am Reaktorende auslaufende Reaktionsgemisch kontinuierlich auf das Festbett des Rohrreaktors zurückpumpt. Die durchschnittliche Verweilzeit betrug nach dieser Verfahrensweise ungefähr 15 Minuten. Es zeigte sich, daß es keinen Vorteil bringt, wenn man zunächst Schwefeltrioxid mit Dimethylsulfat zum reaktionsfähigen Dimethylpyrosulfat umsetzt, bevor dieses mit Chlorbenzol zur Reaktion gebracht wird. Wird nicht auf wäßrige Schwefelsäure, sondern auf Chlorbenzol gefällt, dann erhält man Bis-(4-chlorphenyl)-sulfon in einer Reinheit >99,5 %.

### Beispiele

### Beispiel 1

In eine auf 70°C beheizte, stehende und mit Glaskugeln gefüllte Glasröhre (Länge = 90 cm, Durchmesser = 4 cm) wurden simultan 225 g (2,0 Mol) Chlorbenzol, 126 g (1,0 Mol) Dimethylsulfat und 160 g (2,0 Mol) Schwefeltrioxid mit etwa gleicher Geschwindigkeit innerhalb einer halben Stunde zugetropft. Die Verweilzeit des Reaktionsgemisches in der Festbettkolonne betrug 1 bis 3 Minuten. Das Reaktionsgemisch wurde beim Auslaufen aus dem Reaktor direkt in 1 l einer 5 %igen Schwefelsäure gefällt. Zur Entfernung nicht umgesetzten Chlorbenzoles wurde das Chlorbenzol azeotrop mit Wasser abdestilliert, die Suspension auf Raumtemperatur abgekühlt, abfiltriert, zweimal mit je 500 ml Wasser gewaschen und getrocknet. Nach dem Trocknen erhielt man 164 g (57 %) Bis-(4-chlorphenyl)-sulfon in einer Reinheit von 97 %.

### Beispiel 2

In eine auf 70°C beheizte, stehende und mit Glaskugeln gefüllte Röhre (Länge = 90 cm, Durchmesser = 4 cm) wurden simultan 225 g (2,0 Mol) Chlorbenzol, 126 g (1,0 Mol Dimethylsulfat und 160 g (2,0 Mol) Schwefeltrioxid mit etwa gleicher Geschwindigkeit innerhalb einer halben Stunde zugetropft. Im Gegensatz zu Beispiel 1 wurde das auslaufende Reaktionsgemisch nicht sofort auf verdünnte Schwefelsäure gefällt, sondern kontinuierlich wieder auf die Festbettkolonne zurückgepumpt (Pumpleistung: 2 l/h). Nachdem keine Ausgangskomponente mehr zugetropft wurde, wurde das Reaktionsgemisch noch eine halbe Stunde mit der gleichen Pumpleistung im Kreis geführt und das auslaufende Reaktionsgemisch anschließend auf 1 l einer 5 %igen Schwefelsäure gefällt. Nach der, wie in Beispiel 1 beschriebenen Aufarbeitung wurden 261 g (91 %) Bis-(4-chlorphenyl)-sulfon in einer Reinheit von 97 % erhalten.

### Beispiel 3

Es wurde wie unter Beispiel 2 beschrieben verfahren, jedoch wurde das Reaktionsgemisch nicht auf verdünnte Schwefelsäure, sondern auf 500 ml Chlorbenzol gefällt. Das Fällungsmedium wurde auf Raumtemperatur abgekühlt, filtriert und zwei Mal mit je 200 ml Methanol gewaschen. Nach dem Trocknen wurden 235,3 g (82 %) Bis-(4-chlorphenyl)-sulfon mit einer Reinheit >99,5 % erhalten.

### Beispiel 4

Es wurde wie in Beispiel 1 beschrieben verfahren, jedoch wurden gesinterte Borsäurekugeln (Durchmesser: 4 mm) anstelle von Glaskugeln als Füllkörper verwendet. Nach der üblichen Aufarbeitung und Trocknung erhielt man 187 g (65 %) Bis-(4-chlorphenyl)-sulfon in einer Reinheit von 99 %.

## Patentansprüche

1. Verfahren zur Herstellung von Bis-(4-chlorphenyl)-sulfon, dadurch gekennzeichnet, daß man Schwefeltrioxid, Dimethylsulfat und Chlorbenzol bei 50 bis 100°C gleichzeitig zur Reaktion bringt.

2. Verfahren nach Anspruch 1, dadurch gekennzeichnet, daß man die Reaktion bei 70 bis 90°C durchführt.

3. Verfahren nach Anspruch 1, dadurch gekennzeichnet, daß man Schwefeltrioxid, Dimethylsulfat und Chlorbenzol im Molverhältnis von 0,01:0,005:1 bis 2:1:1 umsetzt.

4. Verfahren nach Anspruch 1, dadurch gekennzeichnet, daß man Schwefeltrioxid, Dimethylsulfat und Chlorbenzol im Molverhältnis von 0,1:0,05:1 bis 1,5:0,8:1 umsetzt.

5. Verfahren nach Anspruch 1, dadurch gekennzeichnet, daß man Schwefeltrioxid, Dimethylsulfat und Chlorbenzol im Molverhältnis von 0,5:0,25:1 bis 1,1:0,6:1 umsetzt.

6. Verfahren nach Anspruch 1, dadurch gekennzeichnet, daß man Schwefeltrioxid, Dimethylsulfat und Chlorbenzol im Molverhältnis von 1:0,5:1 umsetzt.

## Claims

1. A process for preparing bis(4-chlorophenyl) sulfone, which comprises reacting sulfur trioxide, dimethyl sulfate and chlorobenzene all together at from 50 to 100°C.

2. A process as claimed in claim 1, wherein the reaction is carried out at from 70 to 90°C.

3. A process as claimed in claim 1, wherein the molar ratio of sulfur trioxide, dimethyl sulfate and chlorobenzene is from 0.01:0.005:1 to 2:1:1.

4. A process as claimed in claim 1, wherein the molar ratio of sulfur trioxide, dimethyl sulfate and chlorobenzene is from 0.1:0.05:1 to 1.5:0.8:1.

5. A process as claimed in claim 1, wherein the molar ratio of sulfur trioxide, dimethyl sulfate and chlorobenzene is from 0.5:0.25:1 to 1.1:0.6:1.

6. A process as claimed in claim 1, wherein the molar ratio of sulfur trioxide, dimethyl sulfate and chlorobenzene is 1:0.5:1.

## Revendications

1. Procédé de préparation de bis(4-chlorophényl)sulfone, caractérisé en ce qu'on met en réaction simultanément de l'anhydride sulfurique, du sulfate de diméthyle et du chlorobenzène à 50-100°C.

2. Procédé selon la revendication 1, caractérisé en ce qu'on effectue la réaction à 70-90°C.

3. Procédé selon la revendication 1, caractérisé en ce qu'on fait réagir l'anhydride sulfurique, le sulfate de diméthyle et le chlorobenzène dans un rapport molaire de 0,01:0,005:1 à 2:1:1.

4. Procédé selon la revendication 1, caractérisé en ce qu'on fait réagir l'anhydride sulfurique, le sulfate de diméthyle et le chlorobenzène dans un rapport molaire de 0,1:0,05:1 à 1,5:0,8:1.

5. Procédé selon la revendication 1, caractérisé en ce qu'on fait réagir l'anhydride sulfurique, le sulfate de diméthyle et le chlorobenzène dans un rapport molaire de 0,5:0,25:1 à 1,1:0,6:1.

6. Procédé selon la revendication 1, caractérisé en ce qu'on fait réagir l'anhydride sulfurique, le sulfate de diméthyle et le chlorobenzène dans un rapport molaire de 1:0,5:1.
